# EUROPEAN PATENT SPECIFICATION

(11) **EP 0 398 143 B1**
(45) Date of publication and mention of the grant of the patent: **15.03.1995**
(21) Application number: 90108734.6
(22) Date of filing: 09.05.1990
(51) Int. Cl.: A61K 51/08

(54) **Labeled chemotactic peptides to image focal sites of infection or inflammation**
Markierte chemotaktische Peptide zur Visualisierung von Entzündungs- oder Infektionsstellen
Peptides chimiotactiques marqués pour visualiser des sites d'inflammation ou d'infection

(30) Priority: 09.05.1989 US 349186
(43) Date of publication of application: 22.11.1990
(73) Proprietor: THE GENERAL HOSPITAL CORPORATION, Boston, MA 02114 (US); ORTHO PHARMACEUTICAL CORPORATION, Raritan, NJ 08869 (US)
(72) Inventor: Fischman, Alan Jay, Boston, Massachusetts 02114 (US); Rubin, Robert Harold, Brookline, Massachusetts 02146 (US); Strauss, Harry William, Newton, Massachusetts 02159 (US); Fuccello, Anthony Joseph, Princeton, New Jersey 08540 (US); Kroon, Daniel John, Bridgewater, New Jersey 08807 (US); Riexinger, Douglas James, Flemington, New Jersey 08822 (US)
(74) Representative: VOSSIUS & PARTNER

(56) References cited:
- WO-A-87/04351
- Proc. Natl. Acad. Sci. USA, vol. 84, Nov. 1987, pp. 7967-7971, Washington, US; A. ROT et al.: "A series of six ligands for the human formyl peptide receptor: Tetrapeptides with high chemotactic potency and efficacy"
- Journal of Biological Chemistry, vol. 256, no. 1, 10th January 1983, pp. 649-654, Baltimore, US; M. SCHMITT et al.: "Photoaffinity labeling of the N-formyl peptide receptor binding site of intact human polymorphouclear leukocytes"
- Science, vo. 209, 11th July 1980, pp. 297-297, AAAS, Washington, US; B.A. KHAW et al.: "Myocardial infarct imaging of antibodies to canine cardiac myosin with indium-111-diethylenetriamine pentaacetic acid"

## Description

This invention is directed to peptides for diagnosing and treating sites of infection and inflammation in an individual.

Inflammation occurs in response to various forms of tissue damage. This tissue damage can result from microbial invasion, autoimmune processes, tissue or organ allograft rejection, or such injurious external influences as heat, cold, radiant energy, electrical or chemical stimuli, or mechanical trauma. Whatever the cause of bodily site, the ensuing inflammatory response is quite similar, consisting of a complicated set of functional and cellular adjustments, involving the changes in microcirculation, movement of fluids, and influx and activation of inflammatory cells (leukocytes). This response pattern constitutes an important part of innate host defense mechanisms against infection, and although it carries the "cost" of additional tissue damage resulting from the inflammatory process itself, it ultimately promotes the subsequent repair process.

Upon microbial infection, or following certain forms of tissue damage, soluble chemical substances are elaborated which initiate the inflammatory response cascade, consisting of a complex series of events.

Blood flow in the inflammatory site is increased, and in response to increased capillary permeability, there is an efflux of fluid from the blood. This localized exudation of fluid at the site of injury includes plasma proteins that normally leave the capillaries at a relatively low rate. Leukocytes also leave the capillaries into the inflammation site, both by passive and active processes. The inflammatory cellular exudate initially consists primarily of polymorphonuclear (PMN) leukocytes (also termed neutrophils or granulocytes). Subsequently, monocytes, lymphocytes, and plasma cells can be found in the inflammatory infiltrate. These leukocytes produce a variety of mediators that control the extent and duration of the inflammatory response, and bear on their surface a variety of receptors which can bind and respond to the chemical mediators and other proteins present in the inflammatory fluid. Such receptor-mediator interactions are important in controlling leukocyte function within the inflammatory site.

One such class of inflammatory mediators are the chemotactic factors, also called chemoattractants, which have the capacity to induce the directed migration of PMNs, and macrophages (see for general review and discussion: Roitt, I., et al., IMMUNOLOGY, Gower Medical Pub., London 1985). Many species of bacteria can produce one or more types of chemotactic molecules, either small peptides, larger proteins or lipids (Klein, J., IMMUNOLOGY: THE SCIENCE OF SELF-NONSELF DISCRIMINATION, Wiley Interscience, NY, 1982).

For example, E. coli bacteria are known to produce potent chemotactic molecules which have as their active components small, heterogeneous peptides with blocked amino groups. Such peptides have been synthesized and shown to be potent chemoattractants for PMNs. The best known of these chemotactic peptides are N-formyl-methionyl-leucyl-phenylalanine (f-Met-Leu-Phe) and some related tetrapeptides (Schiffman, E., et al., Proc. Natl. Acad. Sci. USA 72:1059-1062 (1975); Schiffman et al., U.S. Patent 4,427,660 (1984)). Structure-function studies of these peptides suggested the presence of stereo-specific receptors on the surface of target cells (Becker. E.L., Am. J. Pathol. 85:385-394 (1976)); such receptors were subsequently detected using radiolabeled formyl peptides as ligands (Williams. L.T., et al., Proc. Natl. Acad. Sci. USA 74:1204-1208 (1977)).

Subsequently, a chemotactic peptide was synthesized with the structure N-Formyl-Nle-Leu-Phe-Nle-Tyr-Lys, which could be labeled to a higher specific radioactivity with ¹²⁵I at the Tyr residue, and was shown to bind strongly to receptors on human neutrophils (Niedel, J., et al., J. Biol. Chem. 254:10700-10706 (1979)).

Bacterial cells can also induce host cells to produce chemotactic factors, e.g., by activating components of the host's complement system, giving rise to the local production of the chemotactic molecules, C5a and C5b67. Once an immune response ensues in the host, IgG antibody molecules may also act as chemotactic factors, as can molecules produced by activated T lymphocytes. Different chemotactic molecules have selectivity for various PMNs (e.g. neutrophils, basophils, eosionphils), mast cells, monocytes/macrophages, or lymphocytes, based on the presence of the appropriate receptors on the inflammatory cell type.

Subsequently, further experiments employing chemotactic peptides have been conducted.

Schmitt, M. et al., (J. Biol. Chem. 256: 649-654 (1983)) demonstrated photoaffinity labeling of the N-formyl peptide receptor binding site of intact human polymorphonuclear leukocytes. In this approach, radioiodinated NPH ((4'-azido-2'-nitrophenylamino)hexanoyl) derivatives of the chemotactic peptide N-formyl-norleucyl-leucyl-phenylalanin-norleucyl-tyrosyl-lysine have been used.

Rot, A. et al., (Proc. Natl. Acad. Sci. USA 84: 7967 - 7971 (1987)) disclose a series of six ligands for the human formyl peptide receptor: tetrapeptides with high chemotactic potency and efficacy. The authors describe in said publication the use of fluorescein isothiocyanate-labeled fMet-Leu-Phe-Lys peptides.

The identification and characterization of the sites of infection and inflammation are important for clinical diagnosis in human and veterinary medicine. In the case of early localized infections, it is frequently necessary to search for "hidden sites of inflammation" in individuals who present with clinical syndromes no more specific than fever and weight loss. Similarly, in patients with autoimmune diseases such as rheumatoid arthritis or in recipients rejecting tissue or organ allografts, the capacity to identify inflammatory sites, define their extent, and monitor changes following initiation of therapy is important for effective clinical area.

Not surprisingly, then, much effort has been expended and many techniques developed in an attempt to identify the site(s) and assess the extent of the inflammatory process. These techniques include conventional x-ray techniques, computerized axial tomographic scanning (CAT scanning), and a variety of radionuclide scans. (Sutton, A Textbook of Radiology and Imaging, 3rd Ed., Churchill Livingston, 1980; Clinical Nuclear Medicine, Maysey et al., ed., W.B. Sanders, 1983.) Examples of techniques for radionuclide scans which have been utilized include:
1. ⁶⁷Gallium, which binds to the plasma protein, transferrin, after injection and tends to localize at sites of chronic inflammation;
2. ¹¹¹Indium labeled granulocytes, which, when reinjected into the host, will accumulate at the site of inflammation;
3. Radiolabeled chelates, which pass into the extracellular fluid and can then accumulate at sites of fluid accumulation; and
4. Thallium scan or so-called first pass radionuclide angiogram to assess ares of increased blood flow.

Thakur, M.L., et al., have extensively analyzed and discussed methods based on neutrophil labeling in vitro (Sem. Nucl. Med. 14:107-117) (1984)). In this approach, an individual's neutrophils are labeled with a gamma emitting radionuclide, ¹¹¹In being the nuclide of choice. Such labeled neutrophils could be used in in vivo kinetic studies and for imaging of inflammatory foci. One disadvantage of this technique is the need to remove and isolate neutrophils prior to their labeling in vitro.

Zoghbi, S.S. et al., (J. Nuc. Med. 22:p32) (1981)), who labeled the chemotactic peptide f_Met-Leu-Phe with ¹¹¹In using the protein transferrin coupled to the peptide and suggested that this reagent had promise for selectively labeling human neutrophils. While this approach provides a potential solution for the problem of specificity of labeling, it does not suggest a solution for the complication of removing cells from an individual, manipulating them in vitro, and then reinfusing them.

One report describes an attempt to localize sterile abscesses in vivo by direct injection of ¹²⁵I-labeled N-Formyl-Nle-Leu-Phe-Nle-Tyr-Lys into rabbits (Jiang, M.-S. et al., Nucl.-Med. 21:110-113 (1982)). While achieving effective labeling ratios (abscess-to-muscle), use of this agent caused transient neutropenia followed by rebound neutrophilia. The authors recognized that further development was needed to avoid the side effects of neutropenia/neutrophilia and make this method clinically useful.

Whereas the above techniques may provide useful information, they result in an unacceptably high frequency of both false positive and false negative results, require an unacceptable amount of handling and processing, or are accompanies by unacceptable side effects. Thus, there is a recognized need in the art for more direct, more sensitive and more specific methods for detecting and localizing sites of injection or inflammation, particularly techniques that could be performed serially to assess the response to therapy over time.

The present invention relates to the embodiments characterized in the claims including a substantially non-invasive method for imaging sites of infection or inflammation.

The inventors have found that detectably labeled chemotactic peptides injected systematically into animals accumulate at sites of local infection.

The present invention thus relates to an in vivo method of detecting a site or sites of infection or inflammation in an individual. This method comprises administering to the individual a detectably labeled chemotactic peptide wherein the peptide substantially accumulates at the site of infection or inflammation. but does not accumulate at uninfected, non-inflamed sites. The invention also includes the various chemotactic peptides to which appropriate chelating molecules and detectable labels have been conjugated.

Figure 1 is a graph demonstrating the results of an assay of the binding of four chemotactic peptides, conjugated with DTPA, to human neutrophils. The assay is a competitive binding assay in which [³H]For-MLF is displaced by increasing concentrations on unlabeled peptide.

Figure 2 is a graph demonstrating the results of an assay of four chemotactic peptides, conjugated to DTPA, for stimulation of superoxide production by human neutrophils. MLF was included in the assay for comparison.

The terms "inflammation" or "site of inflammation" are used to denote conditions and their locations which occur in an individual due to tissue damage, regardless of the underlying cause or etiology. This tissue damage can result from microbial invasion, auto-immune processes, tissue or organ allograft rejection, or such injurious external influences as heat, cold, radiant energy, electrical or chemical stimuli, or mechanical trauma. Whatever the cause or bodily site, the ensuing inflammatory response is quite similar, consisting of a complicated set of functional and cellular adjustments, involving the changes in microcirculation, movement of fluids, and influx and activation of inflammatory cells (leukocytes).

The term "infection" denotes invasion by bacteria, viruses, fungi, protozoa, and other microorganisms.

The term "individual" is meant to include both animals and humans.

In detecting an in vivo site of infection or inflammation in an individual, the detectably labeled chemotactic peptide is advantageously given in a dose which is diagnostically effective. The term "diagnostically effective" means that the amount of detectably labeled chemotactic peptide administered is sufficient to enable detection of the site of infection or inflammation when compared to the background signal.

Generally, the dosage of detectably labeled chemotactic peptide for diagnosis will vary depending on considerations such as age, condition, sex, and extent of disease in the patient, counterindications, if any, and other variables, to be adjusted by the individual physician. Dosage can vary from 0.01 »g/kg to 2,000 »g/kg, preferably 0.1 »g/kg to 1,000 »g/kg.

The term "chemotactic peptide" as used in this invention is meant to include molecules having the following general structure:

N - X - Y - Leu - Phe - Z - W

wherein:
- X: is a bulky group;
- Y: is Met or Nle;
- Z: is a bond or a spacer sequence; and
- W: is a detectable label or a substituent for attaching a label or for attachment to a carrier.

Further chemotactic peptides of the present invention are characterized in the attached set of claims.

A particular preferred embodiment is the peptide having the formula N-formyl-Nle-Leu-Phe-Gly-Gly-Gly-O-Succinyl-SO₃.

At the Z and W positions either D or L amino acids can be used.

Examples for W include the labeling substituent N-^{epsilon}DTPA-lysine or N-^{epsilon}(DTPA)-putrescine and, where W is the substituent for attachment to a carrier, -O-Succinyl-SO₃.

Examples of suitable compounds include, but are not limited to:
For-Nle-Leu-Phe-Nle-Tyr-Lys - DTPA
For-Met-Leu-Phe-Pu - DTPA
For-Nle-Leu-Phe-Lys - DTPA
For-Nle-Leu-Phe-Lys(NH₂) - DTPA
For-Met-Leu-Phe - Lys - DTPA
For-Met-Leu-Phe - D-Lys (NH₂) - DTPA
Ac-Nle-Leu-Phe-Lys(NH₂) - DTPA
The term "diagnostically labeled" means that the chemotactic peptide has attached to it a diagnostically detectable label.

There are many different labels and methods of labeling known to those of ordinary skill in the art. Examples of the types of labels which car, be used in the present invention include radioactive isotopes, paramagnetic isotopes, and compounds which can be imaged by positron emission tomography (PET). Those of ordinary skill in the art will know of other suitable labels for binding to the chemotactic peptides used in the invention, or will be able to ascertain such, using routing experiments. Furthermore, the binding of these labels to the chemotactic peptide can be done using standard techniques common to those of ordinary skill in the art.

For diagnostic in vivo imaging, the type of detection instrument available is a major factor in selecting a given radionuclide. The radionuclide chosen must have a type of decay which is detectable by a given type of instrument. In general, any conventional method for visualizing diagnostic imaging can be utilized in accordance with this invention.

Another important factor in selecting a radionuclide in in vivo diagnosis is that the half-life or a radionuclide be long enough so that it is still detectable at the time of maximum uptake by the target issue, but short enough so that deleterious radiation of the host is minimized. In one preferred embodiment, a radionuclide used for in vivo imaging does not emit particles, but produces a large number of photons in a 140-200 keV range, which may be readily detected by conventional gamma cameras.

For in vivo diagnosis, radionuclides may be bound to chemotactic peptide either directly or indirectly by using an intermediary functional group. Intermediary functional groups which are often used to bind radioisotopes which exist as metallic ions to chemotactic peptides are the chelating agents, diethylene triamine pentaacetic acid (DTPA) and ethylene diamine tetracetic acid (EDTA). Examples of metallic ions which can be bound to chemotactic peptides are ^{99m}Tc, ¹²³I, ¹¹¹In, ¹³¹I, ⁹⁷Ru, ⁶⁷Cu, ⁶⁷Ga, ¹²⁵I, ⁶⁸Ga, ⁷²As, ⁸⁹Zr, and ²⁰¹Tl.

In addition to identifying and characterizing sites of local infection and inflammation, the method of the invention can be used to monitor the course of inflammation in an individual. Thus, by measuring the increase or decrease in the size or number of inflammatory sites it is possible to determine whether a particular therapeutic regimen aimed at ameliorating an infection or other cause of the inflammatory process, or detected to the inflammatory process itself, is effective.

In another embodiment the invention is used for diagnosing the specific underlying cause of the inflammation at the site. In this method an individual suspected of having an inflammatory site is first administered a diagnostically effective amount of chemotactic peptide, as previously described, and is then imaged to determine the presence and location of said site.

In another embodiment, the chemotactic peptides of this invention are "therapeutically conjugated" and used to deliver the therapeutic agent to the site of infection or inflammation. The term "therapeutically conjugated" means that the chemotactic peptide is conjugated to a therapeutic agent. The therapeutic agents used in this manner are directed either to the underlying cause of the inflammation, such as for example, the infectious organisms or a tumor, or to components of the inflammatory process themselves. Examples of agents used to treat inflammation are the steroidal and non-steroidal anti-inflammatory drugs. Many of the non-steroidal anti-inflammatory drugs inhibit prostaglandin synthesis.

Other therapeutic agents which can be coupled to the chemotactic peptides according to the method of the invention are drugs, radioisotopes, lectins, toxins, and antimicrobial agents. The therapeutic dosage administered is an amount which is therapeutically effective, and will be known to one of skill in the art. The dose is also dependent upon the age, health, and weight of the recipient, kind of concurrent treatment, if any, frequency of treatment, and the nature of the effect desired, such as, for example, anti-inflammatory effects or anti-bacterial effect.

Lectins are proteins, often derived from plants, that bind to carbohydrates. Many lectins are also able to agglutinate cells and several stimulate lymphocytes. Ricin is a toxic lectin which has been used therapeutically by binding the alpha-chain, which is responsible for toxicity, to an antibody molecule to enable site-specific delivery of the toxic effect. In an embodiment of this invention, ricin alpha-chain is conjugated to a chemotactic peptide.

Toxins are poisonous substances produced by plants, animals, and microorganisms that, in sufficient dose, can be lethal. Diphtheria toxin, a protein produced by Corynebacterium diphtheriae, consists of separable alpha and beta subunits. The toxic component can be bound to a chemotactic peptide and used for site-specific delivery to the site of an injection or inflammatory response.

Examples of radioisotopes which can be bound to the chemotactic peptide for therapeutic purposes, used according to the method of the invention, are ¹²⁵I, ¹³¹I, ⁹⁰Y, ⁶⁷Cu, ²¹⁷Bi, ²¹¹At, ²¹²Pb, ⁴⁷Sc, and ¹⁰⁹Pd.

Anti-microbials are substances which inhibit such infectious microorganisms as bacteria, viruses, fungi, and parasites (Goodman, A.G., et al., 1985, supra), and can be any of those known to individuals of ordinary skill in the art.

Other therapeutic agents which can be coupled to the chemotactic peptides or specific antibodies used according to the method of the invention are known, or can be easily ascertained, by those of ordinary skill in the art.

Preparations of the imaging chemotactic peptides or therapeutically-conjugated chemotactic peptides for parenteral administration include sterile aqueous or non-aqueous solutions, suspensions, and emulsions. Examples of non-aqueous solvents are propyleneglycol, polyethyleneglycol, vegetable oil such as olive oil, and injectable organic esters such as ethyloleate. Aqueous carries include water, alcoholic/aqueous solutions, emulsions or suspensions, including saline and buffered media, parenteral vehicles including sodium chloride solution, RInger's dextrose, dextrose and sodium chloride, lactated Ringer's, or fixed oils. Intravenous vehicles include fluid and nutrient replenishers, such as those based on Ringer's dextrose, and the like. Preservatives and other additives may also be present, such as, for example, antimicrobials, anti-oxidants chelating agents, and inert gases and the like. See, generally, Remington's Pharmaceutical Science, 16th ed., Mac Eds, 1980.

Preparations of the imaging chemotactic peptides or therapeutically-conjugated chemotactic peptides of the present invention may be administered by any means that achieve their intended purpose. For example, administration may be by parenteral, including subcutaneous, intravenous, intramuscular, intraperitoneal, transdermal, or buccal routes. Alternatively, or concurrently, administration may be by the oral route. A preferred route of administration of the detectably labeled chemotactic peptides for imaging is the intravenous route. The chemotactic peptide can be administered in a single bolus, or by gradual perfusion, which is preferably intravenous and uses peristaltic means to accomplish the gradual perfusion.

This invention can be utilized to detect infection or inflammation at a wide variety of body sites including, but not limited to, muscle, vascular walls, abdomen, pelvis, bone, joint or lung.

This invention is utilized to diagnose infection by any of a number of microorganisms, or inflammation caused by such infection, or by trauma, autoimmune processes or tumors.

The invention is also useful as a means to evaluate the efficacy of, and responses to, therapeutic treatment of infection or inflammation.

The above disclosure generally describes the present invention.

### EXAMPLE 1

### IMAGING OF FOCAL SITES OF EXPERIMENTAL BACTERIAL INFECTION

The chemotactic peptide N-formyl-Nle-Leu-Phe-Nle-Tyr-Lys was synthesized using standard solid phase methods well-known in the art. The epsilon amino group of the C-terminal lysine was modified with DTPA using a cyclic anhydride. The EC₅₀ of the nascent peptide and the DTPA derivative were nearly identical, approximately 10-⁹ M. (Similar results were obtained with all peptides tested). This indicated that the derivatization with DTPA did not affect the biological activity of the peptide. This result was very surprising, since one would expect that introducing the highly negatively charged DTPA group would markedly alter the properties of such small peptides. The peptide was readily radiolabeled with ¹¹¹In.

Six Sprague-Dawley rats were experimentally infected by the injection of 10⁸ viable E. coli into their left thighs. Approximately 100 »Ci of the labeled peptide (5-10 »g) was injected 24 hours after infection. Serial gamma camera images revealed localization of radioactivity at the site of infection with peak accumulation occurring at 1 hour (target-tO-background ration was about 4.0). By 2 hours, the level of activity had decreased in intensity and by 24 hours, the lesion was barely discernible.

These results establish the utility of this new agent for imaging focal sites of inflammation in an animal mode of infection.

### EXAMPLE 2

### BIODISTRIBUTION OF LEUKOCYTE BINDING AND BIOACTIVITY OF CHEMOTACTIC PEPTIDES

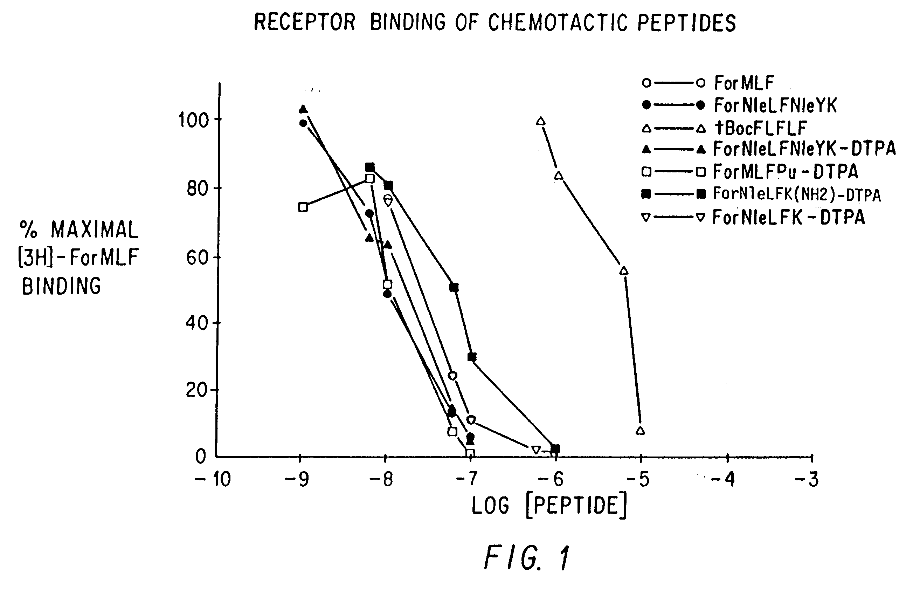

Leukocytic Binding: Four chemotactic peptides conjugated with DTPA were assayed for binding to human neutrophils by a competitive binding assay in which [³H]For-MLF was displaced by increasing concentrations of unlabeled peptide (Babior, B.M. et al., J. Clin. Invest. 52:741 (1973)). The results are shown in Figure 1. Three non-DTPA derivatized peptides were included in the assay for comparison.

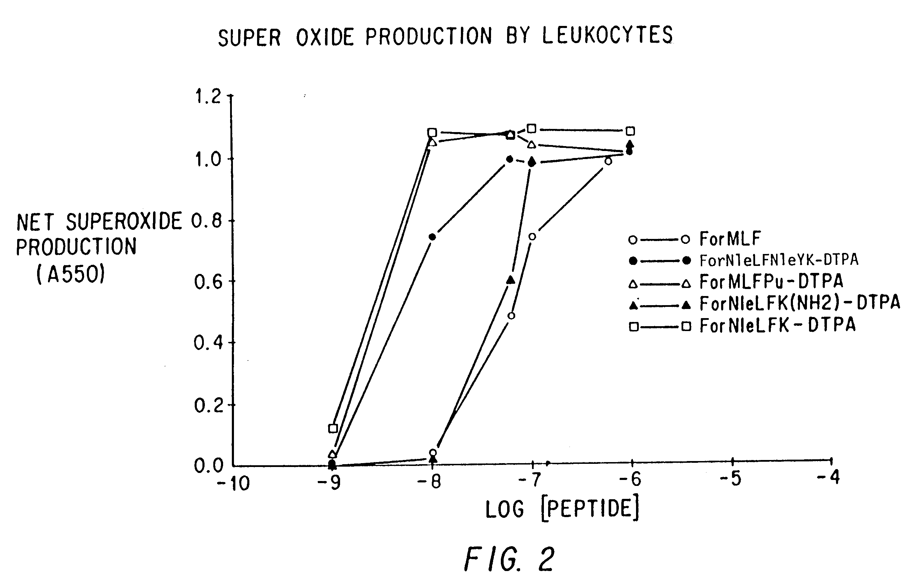

SOD Production: Four chemotactic peptides conjugated with DTPA were assayed for stimulation of superoxide production by human neutrophils (Pike, M.C. et al., Methods in Enzymol. 162:236 (1988)). These results are shown in Figure 2. MLF was included in the assay for comparison purposes.

In Vivo Neutropenia: Two chemotactic peptides conjugated with DTPA were administered to rats at a dose approximately 10-fold higher than the standard imaging dose. Peripheral blood was collected (via tail vein) at 5 min pre-injection and at 1, 2, 5, 10, 15 and 30 minutes post-injection. In all animals studied, there was no significant induction of neutropenia.

While this invention has been described in connection with specific embodiments thereof, it will be understood that it is capable of further modifications. This application is intended to cover any variations, uses, or adaptations of the inventions set forth as follows in the scope of the appended claims.

## Claims (Claims for the following Contracting State(s): AT, BE, CH, DE, DK, FR, GB, IT, LI, LU, NL, SE)

1. A detectably labeled chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a labeling substituent selected from the group consisting of N-^{epsilon} DTPA-lysine and N-^{epsilon} DTPA-putrescine,
wherein said detectable label is bound indirectly to said chemotactic peptide via DTPA.

2. The chemotactic peptide of claim 1, wherein said detectable label comprises a member selected from the group consisting of a radioactive isotope, a paramagnetic isotope, and a compound which can be imaged by PET.

3. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a labeling substituent selected from the group consisting of N-^{epsilon} DTPA-lysine and N-^{epsilon} DTPA-putrescine.

4. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is O-Succinyl-SO₃.

5. The chemotactic peptide according to claim 4 wherein said peptide is N-formyl-Nle-Leu-Phe-Gly-Gly-Gly-O-Succinyl-SO₃.

6. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a radioisotope, wherein said radioisotope is attached to said chemotactic peptide via EDTA.

7. The chemotactic peptide of claim 2 or 6, wherein said radioactive isotope is ¹¹¹In or ^{99m}Tc.

8. The chemotactic peptide according to any one of claims 1 to 3, which is
For-Nle-Leu-Phe-Nle-Tyr-Lys-DTPA;
For-Met-Leu-Phe-Pu-DTPA;
For-Nle-Leu-Phe-Lys-DTPA;
For-Nle-Leu-Phe-Lys(NH₂)-DTPA;
For-Met-Leu-Phe-Lys-DTPA;
For-Met-Leu-Phe-D-Lys(NH₂)-DTPA, or
Ac-Nle-Leu-Phe-Lys(NH₂)-DTPA.

9. A composition containing the chemotactic peptide of any one of claims 1 to 8.

10. The composition of claim 9 which is a therapeutic or diagnostic composition optionally containing in addition a pharmacologically acceptable carrier and/or diluent.

11. Use of the chemotactic peptide of any one of claims 1 to 8 for the preparation of a pharmaceutical composition wherein said composition is prepared in a form suitable to treat a localized site of infection or inflammation in an individual by:
(a) administering to said individual a dose of said composition containing a therapeutically effective amount of said chemotactic peptide is which is therapeutically conjugated; and
(b) accumulating said therapeutically conjugated chemotactic peptide at said site which is infected or inflamed.

12. The chemotactic peptide of any one of claims 1 to 8 for use in a method of detecting a site of infection or inflammation in an individual which comprises:
(a) administering to said individual a diagnostically effective amount of said chemotactic peptide which is detectably labeled;
(b) accumulating said detectably labeled chemotactic peptide at said site which is infected or inflamed; and
(c) detecting said detectably labeled chemotactic peptide.

13. The use of claim 11 or the chemotactic peptide of claim 12 wherein said administration is parenteral.

14. The embodiment according to claim 13, wherein said parenteral administration is by intradermal, subcutaneous, intramuscular, intraperitoneal or intravenous injection.

15. The embodiment according to claim 13, wherein said administration is by gradual perfusion.

16. The embodiment of claim 15, wherein said gradual perfusion is by the intravenous route using peristaltic means.

17. The embodiment of any one of claims 11 to 16, wherein said individual is a human.

## Claims (Claims for the following Contracting State(s): ES)

1. A method for the preparation of a detectably labeled chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a labeling substituent selected from the group consisting of N-^{epsilon} DTPA-lysine and N-^{epsilon} DTPA-putrescine,
wherein the method comprises reacting the epsilon amino group of the Lys group of N-X-Y-Leu-Phe-Z-Lys or the epsilon amino group of the putrescine group of N-X-Y-Leu-Phe-Z-Putrescine with DTPA, and then reacting the DTPA moiety with a detectable label.

2. The method of claim 1, wherein said detectable label comprises a member selected from the group consisting of a radioisotope, a paramagnetic isotope, and a compound which can be imaged by PET.

3. A method for the preparation of a chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is O-Succinyl-SO₃,
wherein the method comprises modifying N-X-Y-Leu-Phe-Z by adding O-Succinyl-SO₃ to the carboxyl group of the terminal glycine residue or the tyrosine residue.

4. The method according to claim 3, wherein said peptide is N-formyl-Nle-Leu-Phe-Gly-Gly-Gly-O-Succinyl-SO₃.

5. A method for the preparation of a chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a radioisotope, wherein said radioisotope is attached to said chemotactic peptide via EDTA,
wherein the method comprises reacting N-X-Y-Leu-Phe-Z with EDTA as an intermediary functional group and reacting the EDTA with a radioisotope.

6. The method of claim 2 or 5, wherein said radioisotope is ¹¹¹In or ^{99m}Tc.

7. The method of claim 1 or 2, wherein said chemotactic peptide is:
For-Nle-Leu-Phe-Nle-Tyr-Lys-DTPA,
For-Met-Leu-Phe-Pu-DTPA,
For-Nle-Leu-Phe-Lys-DTPA,
For-Nle-Leu-Phe-Lys(NH₂)-DTPA,
For-Met-Phe-Lys-DTPA,
For-Met-Leu-Phe-D-Lys(NH₂)-DTPA, or
Ac-Nle-Leu-Phe-Lys-(NH₂)-DTPA.

8. A method for the preparation of a composition containing the chemotactic peptide prepared according to any one of claims 1 to 7.

9. The method of claim 8 wherein said composition is a therapeutic or diagnostic composition optionally containing in addition a pharmacologically acceptable carrier and/or diluent.

## Claims (Claims for the following Contracting State(s): GR)

1. A detectably labeled chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a labeling substituent selected from the group consisting of N-^{epsilon} DTPA-lysine and N-^{epsilon} DTPA-putrescine,
wherein said detectable label is bound indirectly to said chemotactic peptide via DTPA.

2. The chemotactic peptide of claim 1, wherein said detectable label comprises a member selected from the group consisting of a radioactive isotope, a paramagnetic isotope, and a compound which can be imaged by PET.

3. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a labeling substituent selected from the group consisting of N-^{epsilon} DTPA-lysine and N-^{epsilon} DTPA-putrescine.

4. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is O-Succinyl-SO₃.

5. The chemotactic peptide according to claim 4 wherein said peptide is N-formyl-Nle-Leu-Phe-Gly-Gly-Gly-O-Succinyl-SO₃.

6. A chemotactic peptide comprising a peptide having the general formula:
N-X-Y-Leu-Phe-Z-W
wherein:
X is a formyl, acetyl, or a t-Boc group;
Y is Met or Nle;
Z is a bond or a member selected from the group consisting of -Gly-Gly-Gly- and -Nle-Tyr; and
W is a radioisotope, wherein said radioisotope is attached to said chemotactic peptide via EDTA.

7. The chemotactic peptide of claim 2 or 6, wherein said radioactive isotope is ¹¹¹In or ^{99m}Tc.

8. The chemotactic peptide according to any one of claims 1 to 3, which is
For-Nle-Leu-Phe-Nle-Tyr-Lys-DTPA;
For-Met-Leu-Phe-Pu-DTPA;
For-Nle-Leu-Phe-Lys-DTPA;
For-Nle-Leu-Phe-Lys(NH₂)-DTPA;
For-Met-Leu-Phe-Lys-DTPA;
For-Met-Leu-Phe-D-Lys(NH₂)-DTPA, or
Ac-Nle-Leu-Phe-Lys(NH₂)-DTPA.

9. A composition containing the chemotactic peptide of any one of claims 1 to 8.

## Patentansprüche (Patentansprüche für folgende(n) Vertragsstaat(en): AT, BE, CH, DE, DK, FR, GB, IT, LI, LU, NL, SE)

1. Nachweisbar markiertes chemotaktisches Peptid, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist, und
W ein Markierungssubstituent ist, ausgewählt aus N-^{epsilon} -DTPA-Lysin und N-^{epsilon}-DTPA-Putrescin,
wobei der nachweisbare Marker indirekt über DTPA an das chemotaktische Peptid gebunden ist.

2. Chemotaktisches Peptid nach Anspruch 1, wobei der nachweisbare Marker ein radioaktives Isotop, ein paramagnetisches Isotop oder eine Verbindung umfaßt, die durch PET aufgenommen werden kann.

3. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist, und
W ein Markierungssubstituent ist, ausgewählt aus N-^{epsilon}-DTPA-Lysin und N-^{epsilon}-DTPA-Putrescin.

4. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist;
Y Met oder Nle darstellt;
Z eine Bindung oder -Gly-Gly-Gly- oder -Nle-Tyr- ist, und
W die Gruppe O-Succinyl-SO₃ darstellt.

5. Chemotaktisches Peptid nach Anspruch 4, wobei das Peptid N-Formyl-Nle-Leu-Phe-Gly-Gly-Gly-O-Succinyl-SO₃ ist.

6. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist, Y Met oder Nle darstellt,
Z eine Bindung oder -Gly-Gly-Gly- oder -Nle-Tyr- ist, und
W ein Radioisotop darstellt, wobei das Radioisotop über EDTA an das chemotaktische Peptid gebunden ist.

7. Chemotaktisches Peptid nach Anspruch 2 oder 6, wobei das radioaktive Isotop ¹¹¹In oder ^{99m}Tc ist.

8. Chemotaktisches Peptid nach einem der Ansprüche 1 bis 3, nämlich
For-Nle-Leu-Phe-Nle-Tyr-Lys-DTPA;
For-Met-Leu-Phe-Pu-DTPA;
For-Nle-Leu-Phe-Lys-DTPA;
For-Nle-Leu-Phe-Lys(NH₂)-DTPA;
For-Met-Leu-Phe-Lys-DTPA;
For-Met-Leu-Phe-D-Lys(NH₂)-DTPA, oder
Ac-Nle-Leu-Phe-Lys(NH₂)-DTPA.

9. Mittel, enthaltend das chemotaktische Peptid nach einem der Ansprüche 1 bis 8.

10. Mittel nach Anspruch 9, das ein therapeutisches oder diagnostisches Mittel ist, das gegebenenfalls zusätzlich einen pharmakologisch verträglichen Träger und/oder Verdünnungsmittel enthält.

11. Verwendung des chemotaktischen Peptids nach einem der Ansprüche 1 bis 8 für die Herstellung eines Arzneimittels, wobei das Mittel in einer Form hergestellt wird, die zur Behandlung einer bestimmten Infektions- oder Entzündungsstelle in einem Individuum geeignet ist, durch:
(a) Verabreichung an das Individuum einer Dosis des Mittels, die eine therapeutisch wirksame Menge des chemotaktischen Peptids enthält, das therapeutisch konjugiert ist; und
(b) Anreicherung des therapeutisch konjugierten chemotaktischen Peptids an der Stelle, die infiziert oder entzündet ist.

12. Chemotaktisches Peptid nach einem der Ansprüche 1 bis 8 zur Verwendung in einem Verfahren zum Nachweis einer Infektions- oder Entzündungsstelle in einem Individuum, umfassend:
(a) Verabreichung an das Individuum einer diagnostisch wirksamen Menge des chemotaktischen Peptids, das nachweisbar markiert ist;
(b) Anreicherung des nachweisbar markierten chemotaktischen Peptids an der Stelle, die infiziert oder entzündet ist; und
(c) Nachweis des nachweisbar markierten chemotaktischen Peptids.

13. Verwendung nach Anspruch 11 oder des chemotaktischen Peptids nach Anspruch 12, wobei die Verabreichung parenteral erfolgt.

14. Ausführungsform nach Anspruch 13, wobei die parenterale Verabreichung durch intradermale, subkutane, intramuskuläre, intraperitoneale oder intravenöse Injektion erfolgt.

15. Ausführungsform nach Anspruch 13, wobei die Verabreichung durch graduelle Perfusion erfolgt.

16. Ausführungsform nach Anspruch 15, wobei die graduelle Perfusion über den intravenösen Weg unter Verwendung von peristaltischen Mitteln erfolgt.

17. Ausführungsform nach einem der Ansprüche 11 bis 16, wobei das Individuum ein Mensch ist.

## Patentansprüche (Patentansprüche für folgende(n) Vertragsstaat(en): ES)

1. Verfahren zur Herstellung eines nachweisbar markierten chemotaktischen Peptids, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist, und
W ein Markierungssubstituent ist, ausgewählt aus N-^{epsilon}-DTPA-Lysin und N-^{epsilon}-DTPA-Putrescin, umfassend die Umsetzung der ε-Aminogruppe des Lys-Restes von N-X-Y-Leu-Phe-Z-Lys oder der ε-Aminogruppe des Putrescin-Restes von N-X-Y-Leu-Phe-Z-Putrescin mit DTPA und dann Umsetzung der DTPA-Einheit mit einem nachweisbaren Marker

2. Verfahren nach Anspruch 1, wobei der nachweisbare Marker ein radioaktives Isotop, ein paramagnetisches Isotop oder eine Verbindung umfaßt, die durch PET aufgenommen werden kann.

3. Verfahren zur Herstellung eines chemotaktischen Peptids, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist,
und
W die Gruppe O-Succinyl-SO₃ darstellt,
umfassend die Modifizierung von N-X-Y-Leu-Phe-Z durch Zufügung von O-Succinyl-SO₃ an die Carboxylgruppe des terminalen Glycin- oder Tyrosinrests.

4. Verfahren nach Anspruch 3, wobei das Peptid N-Formyl-Nle-Leu-Phe-Gly-Gly-Gly-O-Succinyl-SO₃ ist.

5. Verfahren zur Herstellung eines chemotaktischen Peptids, umfassend ein Peptid mit der allgemeinen Formel:
N-X-Y-Leu-Phe-Z-W
in der
X eine Formyl-, Acetyl- oder t-Boc-Gruppe ist,
Y Met oder Nle darstellt,
Z eine Bindung oder -Gly-Gly-Gly- oder -Nle-Tyr- ist, und
W ein Radioisotop darstellt, wobei das Radioisotop über EDTA an das chemotaktische Peptid gebunden ist,
umfassend die Umsetzung von N-X-Y-Leu-Phe-Z mit EDTA als einer intermediären funktionellen Gruppe und Umsetzung des EDTA mit einem Radioisotop.

6. Verfahren nach Anspruch 2 oder 5, wobei das radioaktive Isotop ¹¹¹In oder ^{99m}Tc ist.

7. Verfahren nach Anspruch 1 oder 2, wobei das chemotaktische Peptid
For-Nle-Leu-Phe-Nle-Tyr-Lys-DTPA,
For-Met-Leu-Phe-Pu-DTPA,
For-Nle-Leu-Phe-Lys-DTPA,
For-Nle-Leu-Phe-Lys(NH₂)-DTPA,
For-Met-Leu-Phe-Lys-DTPA,
For-Met-Leu-Phe-D-Lys(NH₂)-DTPA, oder
Ac-Nle-Leu-Phe-Lys(NH₂)-DTPA.
ist.

8. Verfahren zur Herstellung eines Mittels, enthaltend das chemotaktische Peptid hergestellt nach einem der Ansprüche 1 bis 7.

9. Verfahren nach Anspruch 8, wobei das Mittel ein therapeutisches oder diagnostisches Mittel ist, das gegebenenfalls zusätzlich einen pharmakologisch verträglichen Träger und/oder Verdünnungsmittel enthält.

## Patentansprüche (Patentansprüche für folgende(n) Vertragsstaat(en): GR)

1. Nachweisbar markiertes chemotaktisches Peptid, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist, und
W ein Markierungssubstituent ist, ausgewählt aus N-^{epsilon}-DTPA-Lysin und N-^{epsilon}-DTPA-Putrescin,
wobei der nachweisbare Marker indirekt über DTPA an das chemotaktische Peptid gebunden ist.

2. Chemotaktisches Peptid nach Anspruch 1, wobei der nachweisbare Marker ein radioaktives Isotop, ein paramagnetisches Isotop oder eine Verbindung umfaßt, die durch PET aufgenommen werden kann.

3. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist,
Y Met oder Nle bedeutet,
Z eine Bindung oder -Gly-Gly-Gly- oder -Nle-Tyr- ist, und
W ein Markierungssubstituent ist, ausgewählt aus N-^{epsilon}-DTPA-Lysin und N-^{epsilon}-DTPA-Putrescin.

4. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist;
Y Met oder Nle darstellt;
Z eine Bindung oder -Gly-Gly-Gly- oder -Nle-Tyr- ist, und
W die Gruppe O-Succinyl-SO₃ darstellt.

5. Chemotaktisches Peptid nach Anspruch 4, wobei das Peptid N-Formyl-Nle-Leu-Phe-Gly-Gly-Gly-O-Succinyl-SO₃ ist.

6. Chemotaktisches Peptid, umfassend ein Peptid mit der allgemeinen Formel:
N-X-Y-Leu-Phe-Z-W
in der
X eine Formyl-, Acetyl- oder t-Boc-Gruppe ist,
Y Met oder Nle darstellt,
Z eine Bindung oder -Gly-Gly-Gly- oder -Nle-Tyr- ist, und
W ein Radioisotop darstellt, wobei das Radioisotop über EDTA an das chemotaktische Peptid gebunden ist.

7. Chemotaktisches Peptid nach Anspruch 2 oder 6, wobei das radioaktive Isotop ¹¹¹In oder ^{99m}Tc ist.

8. Chemotaktisches Peptid nach einem der Ansprüche 1 bis 3, nämlich
For-Nle-Leu-Phe-Nle-Tyr-Lys-DTPA;
For-Met-Leu-Phe-Pu-DTPA;
For-Nle-Leu-Phe-Lys-DTPA;
For-Nle-Leu-Phe-Lys(NH₂)-DTPA;
For-Met-Leu-Phe-Lys-DTPA;
For-Met-Leu-Phe-D-Lys(NH₂)-DTPA, oder
Ac-Nle-Leu-Phe-Lys(NH₂)-DTPA.

9. Mittel, enthaltend das chemotaktische Peptid nach einem der Ansprüche 1 bis 8.

## Revendications (Revendications pour l'(les) Etat(s) contractant(s) suivant(s): AT, BE, CH, DE, DK, FR, GB, IT, LI, LU, NL, SE)

1. Peptide chimiotactique marqué détectable comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un substituant marqueur choisi dans le groupe constitué par N-^{epsilon} DTPA-lysine et N-^{epsilon} DTPA-putrescine,
dans lequel ledit marqueur détectable est lié indirectement audit peptide chimiotactique par l'intermédiaire de DTPA.

2. Le peptide chimiotactique de la revendication 1, dans lequel ledit marqueur détectable comprend un élément choisi dans le groupe constitué par un isotope radioactif, un isotope paramagnétique, et un composé dont l'image peut être obtenue par tomoscintigraphie (PET).

3. Peptide chimiotactique comprenant un peptide ayant la formule générale
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un substituant marqueur choisi dans le groupe constitué par N-^{epsilon} DTPA-lysine et N-^{epsilon} DTPA-putrescine.

4. Peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est O-Succinyl-SO₃.

5. Le peptide chimiotactique selon la revendication 4, dans lequel ledit peptide est N-formyl-Nle-Leu-Phe-Gly-Gly-Gly-O-Succinyl-SO₃.

6. Peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un radio-isotope, où ledit radio-isotope est rattaché audit peptide chimiotactique par l'intermédiaire d'EDTA.

7. Le peptide chimiotactique de la revendication 2 ou 6, dans lequel ledit isotope radioactif est ¹¹¹In ou ^{99m}Tc.

8. Le peptide chimiotactique selon l'une quelconque des revendications 1 à 3, constitué par :
For-Nle-Leu-Phe-Nle-Tyr-Lys-DTPA ;
For-Met-Leu-Phe-Pu-DTPA ;
For-Nle-Leu-Phe-Lys-DTPA ;
For-Nle-Leu-Phe-Lys(NH₂)-DTPA ;
For-Met-Leu-Phe-Lys-DTPA ;
For-Met-Leu-Phe-D-Lys(NH₂)-DTPA, ou
Ac-Nle-Leu-Phe-Lys (NH₂)-DTPA.

9. Composition contenant un peptide chimiotactique selon l'une quelconque des revendications 1 à 8.

10. La composition de la revendication 9, qui est une composition thérapeutique ou de diagnostic contenant en outre, le cas échéant, un support et/ou un diluant pharmacologiquement acceptables.

11. Utilisation d'un peptide chimiotactique selon l'une quelconque des revendications 1 à 8 dans la préparation d'une composition pharmaceutique, dans laquelle ladite composition est préparée dans une forme adaptée au traitement d'un site localisé d'infection ou d'inflammation chez un individu :
(a) en administrant audit individu une dose de ladite composition contenant une quantité thérapeutiquement efficace dudit peptide chimiotactique qui est thérapeutiquement associé ; et
(b) en accumulant ledit peptide chimiotactique thérapeutiquement associé sur ledit site infecté ou enflammé.

12. Le peptide chimiotactique de l'une quelconque des revendications 1 à 8, utilisé dans un procédé de détection d'un site d'infection ou d'inflammation chez un individu, comprenant :
(a) l'administration audit individu d'une quantité efficace pour le diagnostic dudit peptide chimiotactique qui est marqué et détectable ;
(b) l'accumulation dudit peptide chimiotactique marqué et détectable sur ledit site infecté ou enflammé ; et
(c) la détection dudit peptide chimiotactique marqué détectable.

13. L'utilisation de la revendication 11 ou le peptide chimiotactique de la revendication 12, où ladite administration est parentérale.

14. La réalisation de la revendication 13, dans laquelle ladite administration parentérale se fait par injection intradermique, subcutanée, intramusculaire, intrapéritonéale ou intraveineuse.

15. La réalisation de la revendication 13, dans laquelle ladite administration se fait par perfusion progressive.

16. La réalisation de la revendication 15, dans laquelle ladite perfusion progressive se fait par la voie intraveineuse en utilisant des moyens péristaltiques.

17. La réalisation de l'une quelconque des revendications 11 à 16, dans laquelle ledit individu est un humain.

## Revendications (Revendications pour l'(les) Etat(s) contractant(s) suivant(s): ES)

1. Procédé de préparation d'un peptide chimiotactique marqué détectable comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un substituant marqueur choisi dans le groupe constitué par N-^{epsilon} DTPA-lysine et N-^{epsilon} DTPA-putrescine,
dans lequel le procédé consiste à faire réagir le groupe epsilon amino du groupe Lys de N-X-Y-Leu-Phe-Z-Lys ou le groupe epsilon amino du groupe putrescine de N-X-Y-Leu-Phe-Z-putrescine avec la DTPA, et ensuite à faire réagir la partie DTPA avec un marqueur détectable.

2. Le procédé de la revendication 1, dans lequel ledit marqueur détectable comprend un élément choisi dans le groupe constitué par un radio-isotope, un isotope paramagnétique, et un composé dont l'image peut être obtenue par tomoscintigraphie (PET).

3. Procédé de préparation d'un peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est O-Succinyl-SO₃,
dans lequel le procédé comprend la modification de N-X-Y-Leu-Phe-Z par addition de O-Succinyl-SO₃ au groupe carboxyle du résidu glycine ou tyrosine terminal.

4. Le procédé selon la revendication 3, dans lequel ledit peptide est N-formyl-Nle-Leu-Phe-Gly-Gly-Gly-O-Succinyl-SO₃.

5. Procédé de préparation d'un peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un radio-isotope, où ledit radio-isotope est rattaché audit peptide chimiotactique par l'intermédiaire d'EDTA,
dans lequel le procédé comprend la réaction de N-X-Y-Leu-Phe-Z avec l'EDTA comme groupe fonctionnel intermédiaire, et la réaction de l'EDTA avec un radio-isotope.

6. Le procédé de la revendication 2 ou 5, dans lequel ledit radio-isotope est ¹¹¹In ou ^{99m}Tc.

7. Le procédé de la revendication 1 ou 2, dans lequel ledit peptide chimiotactique est :
For-Nle-Leu-Phe-Nle-Tyr-Lys-DTPA ;
For-Met-Leu-Phe-Pu-DTPA ;
For-Nle-Leu-Phe-Lys-DTPA ;
For-Nle-Leu-Phe-Lys(NH₂)-DTPA ;
For-Met-Leu-Phe-Lys-DTPA ;
For-Met-Leu-Phe-D-Lys(NH₂)-DTPA, ou
Ac-Nle-Leu-Phe-Lys (NH₂)-DTPA.

8. Procédé de préparation d'une composition contenant un peptide chimiotactique préparé selon l'une quelconque des revendications 1 à 7.

9. Le procédé de la revendication 8, dans lequel ladite composition est une composition thérapeutique ou de diagnostic contenant en outre, le cas échéant, un support et/ou un diluant pharmacologiquement acceptables.

## Revendications (Revendications pour l'(les) Etat(s) contractant(s) suivant(s): GR)

1. Peptide chimiotactique marqué détectable comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un substituant marqueur choisi dans le groupe constitué par N-^{epsilon} DTPA-lysine et N-^{epsilon} DTPA-putrescine,
dans lequel ledit marqueur détectable est lié indirectement audit peptide chimiotactique par l'intermédiaire de DTPA.

2. Le peptide chimiotactique de la revendication 1, dans lequel ledit marqueur détectable comprend un élément choisi dans le groupe constitué par un isotope radioactif, un isotope paramagnétique, et un composé dont l'image peut être obtenue par tomoscintigraphie (PET).

3. Peptide chimiotactique comprenant un peptide ayant la formule générale
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un substituant marqueur choisi dans le groupe constitué par N-^{epsilon} DTPA-lysine et N-^{epsilon} DTPA-putrescine.

4. Peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est O-Succinyl-SO₃.

5. Le peptide chimiotactique selon la revendication 4, dans lequel ledit peptide est N-formyl-Nle-Leu-Phe-Gly-Gly-Gly-O-Succinyl-SO₃.

6. Peptide chimiotactique comprenant un peptide ayant la formule générale :
N - X - Y - Leu- Phe- Z - W
dans laquelle
X est un groupe formyle, acétyle ou t-Boc ;
Y est Met ou Nle ;
Z est une liaison ou un élément choisi dans le groupe constitué par -Gly-Gly-Gly et -Nle-Tyr ; et
W est un radio-isotope, où ledit radio-isotope est rattaché audit peptide chimiotactique par l'intermédiaire d'EDTA.

7. Le peptide chimiotactique de la revendication 2 ou 6, dans lequel ledit isotope radioactif est ¹¹¹In ou ^{99m}Tc.

8. Le peptide chimiotactique selon l'une quelconque des revendications 1 à 3, constitué par :
For-Nle-Leu-Phe-Nle-Tyr-Lys-DTPA ;
For-Met-Leu-Phe-Pu-DTPA ;
For-Nle-Leu-Phe-Lys-DTPA ;
For-Nle-Leu-Phe-Lys(NH₂)-DTPA ;
For-Met-Leu-Phe-Lys-DTPA ;
For-Met-Leu-Phe-D-Lys(NH₂)-DTPA, ou
Ac-Nle-Leu-Phe-Lys (NH₂)-DTPA.

9. Composition contenant un peptide chimiotactique selon l'une quelconque des revendications 1 à 8.
